Europäisches Patentamt

European Patent Office

Office européen des brevets

(11) Publication number: **0 547 689 A2**

## EUROPEAN PATENT APPLICATION

(21) Application number: **92203855.9**

(22) Date of filing: **10.12.92**

(51) Int. Cl.⁵: **C07K 7/10, A61K 39/21, C07K 7/08, C07K 7/06**

(30) Priority: **18.12.91 US 811041**

(43) Date of publication of application:
**23.06.93 Bulletin 93/25**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Hannah, John**
**155 Idlebrook Lane**
**Matawan, NJ 07747(US)**
Inventor: **Tolman, Richard L.**
**29 Upper Warren Way**
**Warren, NJ 07059(US)**

(74) Representative: **Barrett-Major, Julie Diane et al**
**Merck & Co., Inc. European Patent Department Terlings Park Eastwick Road Harlow Essex CM20 2OR (GB)**

(54) **Synthetic peptides comprising a cyclic HIV principal neutralizing determinant and a T-cell stimulatory epitope.**

(57) A synthetic peptide comprising a cyclic HIV prinicipal neutralizing determinant and a Hepatitis B T-cell stimulatory epitope raises high titers of HIV neutralizing antibodies in a mammal.

EP 0 547 689 A2

Rank Xerox (UK) Business Services
(3.10/3.6/3.3.1)

## BACKGROUND OF THE INVENTION

Immunogens directed at production of immune responses against human immunodeficiency virus (HIV) are under active development worldwide. Acquired immune deficiency syndrome (AIDS) and other diseases associated with HIV infection are reaching epidemic proportions on a global basis. This invention was made in response to the need for effective immunogens which are able to induce immune responses directed against the etiologic agent of these diseases.

Use of complete viral proteins, such as gp120 or gp160 has met with limited success as a vaccine candidate, as the subunit proteins appear to induce a number of immunological responses, not all of which are beneficial in preventing HIV infection.

Presentation of peptidyl epitopes displayed by the intact virus or its subunit proteins, has also been attempted. Thus, peptide epitopes have been conjugated to carrier proteins or they have been produced as fusion proteins with carrier proteins.

Of particular interest in this regard are peptidyl epitopes derived from the third variable domain of HIV gp120, referred to hereinafter as principal neutralizing determinant (PND) peptides. These are peptide epitopes against which HIV neutralizing antibodies are directed. A preferred subset of the PND sequences are those comprising the sequence Gly Pro Gly or Gly Pro Gly Arg [Seq. ID:1:]. This sequence is found in a great many isolates of HIV in the third variable region of the HIV envelope protein gp120, and appears to be part of an important epitope, proper exposure of which to mammalian immune systems results in induction of HIV neutralizing immune responses.

The above noted approaches hold promise, but the immunogens are complicated to produce, and successful HIV vaccines are not available. Those in clinical study are not conjugate vaccines and do not provide reproducibly high levels of neutralizing antibodies. Experimental conjugate vaccines are difficult to prepare and parameters of loading, sterility, and pyrogenicity are difficult to control. Replacement of the carrier protein with a low molecular weight moiety with known structure and composition to form a hybrid molecule will avoid these problems. Hybrid peptides are easier to prepare, have a more readily determinable composition, and are not subject to the same regulatory requirements as carrier protein conjugates.

A desirable method of producing an effective immunogen would be a completely synthetic route which yelds a low molecular weight compound which is immunogenic. It is also desirable that the synthetic immunogen be capable of priming both B-cell and T-cell mediated immune responses, because HIV is known to multiply by cell-cell fusion (syncytia formation) and also to be present in the bloodstream where antibodies may bind to and neutralize the virus.

T-cell mitogens are substances which induce mitosis of T-cells. Lectins, such as phytohemagglutinin, concanavalin A, pokeweed mitogen, lipopolysaccharide, lectins extractable from peanut, soybean, or pea are all known to exhibit mitogenic effects. Mitogenesis, particularly of T-helper cells, appears to be essential for the generation of antibodies by B-cells in response to antigens of low complexity. It should be obvious to those skilled in the art that known T-cell mitogens may be used to prime B-cells to respond to the cyclic B-cell epitopes in the manner disclosed in this invention.

T-cell mitogens of various types are known, but immunologically effective hybrid peptides comprising cyclic HIV $V_3$-loop sequences have not been reported, nor are hybrid peptides known which comprise a small cyclic HIV B-cell determinant and a hepatitis B T-cell determinant. For example, in WO 91/04051, Fuerst and Koenig described cytotoxic T-lymphocyte epitopes of HIV derived from the NEF, GAG and ENV proteins. However, no information was presented to suggest that the peptides could be used as an immunogen to raise immune responses against HIV.

In WO 91/02544, Girard et al., disclosed a composition containing a hybrid molecule comprising a B-cell epitope of the envelope glycoprotein of a retrovirus and a T-cell epitope from another retroviral protein. The hybrid molecules are linear molecules of about 24-32 amino acids.

In U.S. Patent 4,943,628, Rosen and Warner described HIV derived peptides for T-cell stimulation. The peptides are linear molecules of less than about 60 amino acids derived from gp120.

Palker et al.[J. Immunol 142, 3612 (1989)] and Hart et al. [J. Immunol 145, 2677 (1990)] reported on a hybrid HIV T-cell epitope and HIV B-cell epitope. The B-cell epitope, however, was not cyclized, and the T-cell epitope did not originate from the heterologous Hepatitis B Virus as in the instant invention.

In a field of research unrelated to HIV vaccine development, Milich et al., [J. Immunol. 138, 4457-4465 (1987)], described a series of experiments directed at identification and characterization of T-cell and B-cell recognition sites within the pre-S(1) region of hepatitis B surface antigen p43 (HBsAg/p43). In the course of that work, those researchers identified three sequences, designated p12-21, p94-105 and p106-117, all of which were capable of inducing in vitro proliferation of T-cells primed to larger hepatitis-B specific peptides. However, these sequences were found to be poor stimulators of T-cells in vivo. The p12-21 sequence, whose amino acid sequence is:

Met Gly Thr Asn Leu Ser Val Pro Asn Pro [Seq. ID:2:], was found to function as a T-cell carrier for a synthetic pre-S2 region B-cell determinant. The 10 amino acid sequence was reported to be the minimum sequence required to elicit an efficient pre-S(1)-specific T-cell proliferative response in vitro. However, a longer sequence, p12-32,

Met Gly Thr Asn Leu Ser Val Pro Asn Pro Leu Gly Phe Phe Pro Asp His Gln Leu Asp Pro

[Seq. ID:3:],

and p1-21,

Met Gly Gly Trp Ser Ser Lys Pro Arg Lys Gly Met Gly Thr Asn Leu Ser Val Pro Asn Pro

[Seq. ID:4:], were found to be as effective or more effective than the p12-21 sequence.

Other T-cell stimulatory epitopes, besides this hepatitis B T-cell epitope, may also be used according to the disclosure of this invention, to enhance the efficacy of cyclic HIV PND peptides. Thus, based on the instant disclosure, it would be obvious to those skilled in the art that the T-cell epitope containing peptide, -Thr Lys Ile Ser Asp Phe Gly Ser Phe Ile Gly Phe Lys-NH$_2$ [Seq. ID:13:], (sequence 47-59 of the meningococcal outer membrane protein) used by Boons et al., [Bioorganic & Medicinal Chemistry Letters 1, No. 6, p. 303-308 (1991)] to stimulate an anti-meningococcal polysaccharide immune response, could be used according to this invention.

The instant invention provides a hybrid immunogen comprising a T-cell stimulatory sequence from hepatitis-B pre-S(1) and cyclic HIV principal neutralizing determinant (cPND) sequences, which has the surprising property of being a very good immunogen for raising anti-PND, anti-HIV, or HIV-neutralizing immune responses in vivo.

Hybrids of mitogenic sequences and cyclic V$_3$-derived peptides are prepared by fabrication of the peptide, cyclization as appropriate, and post-synthetic modification. The T-cell mitogenic properties replace the carrier protein function of conventional HIV conjugate vaccines.

## SUMMARY OF THE INVENTION

This invention is a hybrid immunogen comprising a T-cell stimulatory epitope from the pre-S(1) region of HBsAg/p43 and a cyclic human immunodeficiency virus (HIV) principal neutralizing determinant (cPND). In a preferred embodiment of the invention, the T-cell stimulatory epitope comprises the sequence:

Met Gly Thr Asn Leu Ser Val Pro Asn Pro

[Seq. ID:2:], and the HIV PND comprises the sequence:

Gly Pro Gly Arg [Seq. ID:1:]

## DETAILED DESCRIPTION OF THE INVENTION

The instant invention is a hybrid immunogen comprising a T-cell epitope from the pre-S(1) region of HBSAg/p43 and an HIV PND. The hybrid may be prepared by conjugating peptides derived from HBsAg and HIV proteins, by recombinant expression of nucleic acid sequences specifying the hybrid amino acids, or by direct synthesis by methods known in the art. In a preferred embodiment of the invention, the hybrid immunogen is prepared by direct synthesis of the hybrid molecule, wherein the HIV PND is a cyclic peptide.

In one embodiment of the invention, the T-cell epitope comprises the sequence:

Met Gly Thr Asn Leu Ser Val Pro Asn Pro

[Sequence. ID:2:], while the cyclic HIV PND comprises the sequence Gly Pro Gly. In a highly preferred embodiment of the invention, the HIV PND, comprising the Gly Pro Gly sequence, is a cycle formed through a disulfide bond, a thioether bond, an amide bond, or any other practical means of ring closure such that the Gly Pro Gly portion of the molecule is exposed as a prominent feature of the PND loop.

Peptide hybrids of V$_3$-derived sequences and T-cell mitogenic sequences are effective vaccines, where the T-cell mitogenic sequence replaces the carrier function of a conventional conjugate vaccine. The hybrids can be given with or without appropriate adjuvants. An immunologically effective amount of the hybrid should be administered intramuscularly, subcutaneously, intravenously, intraperitoneally, or by any other route found to be efficacious in achieving exposure of the immunogen to the immune responsive system.

By intramuscular administration, a dose of about 0.1 μg to 1 mg and preferably about 10 μg - 500 μg of immunogen is administered, in a pharmaceutically acceptable medium. An adjuvant such as Freunds complete or incomplete, or the Ribi adjuvant may be admixed with the conjugate immunogen prior to administration. One can present the hybrid in an adjuvant comprising liposomes, preferably comprising monophosphoryl lipid A. It is also beneficial to adsorb the immunogen to aluminum hydroxide gel prior to administration. Furthermore, antivirals, immunomodulators, antibacterials or other pharmaceuticals may be co-administered to advantage.

The hybrid is administered intramuscularly at a dose of 10 to 500 $\mu$g/ in a volume of 1 mL, preferably adsorbed to 500 $\mu$g/ml of aluminum hydroxide gel.

Thus, in one preferred embodiment of the invention, the compound cPND566 was prepared by solid phase synthesis (see Example 1), as was cPND224 (See Example 2) and cPND999 (see Example 6). cPND566 and cPND224 were tested and raised high titers of HIV neutralizing antibodies in rabbits. (See Examples 3, 4, and 5).

Other obvious variants on the theme suggested by these compounds naturally come within the scope of this invention. Thus, hybrids wherein the HIV cPND and Hepatitis B epitope are linked through intervening sequences of between 1 to 4 amino acids, which may include a marker amino acid, such as norleucine, naturally fall within this invention as do hybrids wherein the cPND comprising the sequence Gly Pro Gly Arg [Seq. ID:1:] comprises cyclic peptides of between 5 and 40 amino acids, cyclized through any of a number of means known in the art, including disulfide bonds, thioether bonds, or amide bonds.

cPND566

H—Met Gly Thr Asn Leu Ser Val Pro Asn Pro Cys Arg Lys Arg Ile His Ile Gly Pro Gly Arg Ala Phe Tyr Thr Thr Lys Cys—NH2.

[Seq. ID:5:]

cPND244:

H—Met Gly Thr Asn Leu Ser Val Pro Asn Pro Nle Cys His Ile Gly Pro Gly Arg Ala Phe Cys—OH

[Seq. ID:6:]

cPND999:

```
H—Met Gly Thr Asn Leu Ser Val Pro Asn Pro Nle Cys Tyr Asn Lys Arg Lys Arg
                                                 |                          Ile
[Seq. ID:14:]                                 HO—Cys                        His
                                                Gly                         Ile
                                                Ile                         Gly
                                                Ile                         Pro
                                                Asn                         Gly
                                            Lys Thr Thr Tyr Phe Ala Arg
```

A general structure for this invention, therefor, may be shown as [Seq. Id:15:]:

X₁ - Met Gly Thr Asn Leu Ser Val Pro Asn Pro-X₂ X₃ X₄ Gly Pro Gly Arg X₅ X₆ X₇,

wherein:

X₁ is hydrogen,
Met Gly Gly Trp Ser Ser Lys Pro Arg Lys Gly [Seq. Id :11:], or a portion thereof;
Met Gly Thr Asn Leu Ser Val Pro Asn Pro-X₂ X₃ X₄ Gly Pro Gly Arg X₅ X₆ X₇, or a portion thereof;

X₂ is a bond,
Leu Gly Phe Phe Pro Asp His Gln Leu Asp [Seq. Id. :12:], or a portion thereof, or any sequence of 1 to 4 amino acids optionally including a marker amino acid such as norleucine;

$X_3$ is     an amino acid which is bonded to $X_6$ and is preferably cysteine;

$X_4$ is     1 to 25 amino acids long, selected from the sequence of amino acids found in any of the common isolates of HIV gp120, amino terminal and adjacent to the Gly Pro Gly Arg [Seq. Id.:1:] sequence;

$X_6$ is     a bond or 1 to 25 amino acids long, selected from the sequence of amino acids found in any of the common isolates at HIV gp120, carboxy-terminal and adjacent to the Gly Pro Gly Arg [Seq. Id.:1:] sequence;

$X_6$ is     an amino acid which is bonded to X3, and is preferably cysteine;

$X_7$ is     hydroxyl,

          -NH2, or

          between 1 and 10 amino acids long.

This immunogen may be further defined according to the HIV cPND wherein:

$X_1$ is     hydrogen;

$X_2$ is     Nle;

$X_3$ and $X_6$ are Cys; and $X_4$, $X_6$ and $X_7$ are as defined above.

Guidance as to the sequences which are preferred for $X_4$ and $X_6$ is provided by LaRosa et al, Science 249, 932-935 (1990), wherein the sequence of 245 HIV isolates was examined. A consensus sequence for $X_4$ may be defined from that work as truncations or variations of:

Thr Arg Pro Asn Asn Asn Thr Arg Lys Ser Ile His Ile [Seq. Id:7:], while $X_6$ may be defined as truncations or variations of:

Tyr Thr Thr Gly Glu Ile Ile Gly Asp Ile Arg Gln Ala His [Seq. I:8:].

In one preferred embodiment,

$X_4$ is     Arg Lys Arg Ile His Ile, [Seq. Id:9:], and

$X_6$ is     Tyr Thr Thr Lys [Seq. Id:10:].

In another preferred embodiment,

$X_4$ is His Ile, and $X_6$ is a bond.

The following examples are provided to further illustrate the invention, and are not to be construed as limiting the invention.

EXAMPLE 1

Preparation of cPND566:

A. Preparation of the linear 28mer:

The 28mer was assembled on the MILLIGEN #9050 synthesizer, starting from Fmoc-PAL-PEGMBHA resin (0.907 g, 0.200 milliequivalents, MILLIGEN lot PDSTI-91B, 0.22 meq/g.

The Fmoc-PAL linkage was cleaved with 20% piperidine/NMP, and the peptide chain is built on the 2,4,6-alkoxybenzylamine, and finally cleaved with TFA to give the carboxy terminus as an amide.

Reagents were Fmoc Pfp esters, except for Ser and Thr which were dihydrobenzotriazene (Dhbt)esters. The monomers were provided in four fold excess in NMP solvent. Side chain protection was as follows:

Thr, Ser, Tyr     - Bu$^t$ = tert Butyl

Arg               - Mtr = 4 methoxy-2,3,5-trimethylphenyl sulfonyl

His, Lys          - Boc = tert-butyloxycarbonyl

Cys               - Trt = trityl.

Double coupling was provided for Thr$^3$, Ser$^6$, Val$^7$, Pro$^8$, Asn$^9$, Pro$^{10}$, Lys$^{13}$, Ile$^{15}$, Ile$^{17}$ Gly$^{18}$, Pro$^{19}$, Gly$^{20}$, Phe$^{23}$, Tyr$^{24}$, Thr$^{25}$, Thr$^{26}$.

The dry Fmoc-PAL-PEGMBHA resin (0.907 g) was charged to a 1 x 10 cm column, covered with anhydrous NMP and the resin swelled. The machine was programmed for synthesis, the final Fmoc was cleaved, and the column washed with DCM.

The resin was washed out of the column with $CH_2Cl_2$ and dried. The derivatized resin was suspended in 95% TFA/4% ethanadithiol/1% thioanisole (20 ml) and the peptide allowed to cleave from the resin for 15 hours at 23°C. The resin was filtered off and washed with 100% TFA (2 x 10 ml). The combined filtrate was evaporated and residual traces of TFA and scavengers were extracted by trituration of the viscous gum in ether (3 x 10 ml). The insoluble product was recovered by filtration and dried to yield an almost colorless powder (494 mg).

About 50 $\mu$g of the powder was dissolved in 50 $\mu$l aq. 0.1% TFA/20% $CH_3CN$, and a 6 $\mu$l sample was analyzed on a Vydac $C_{18}$ reverse phase column (0.46 x 25.0 cm) equilibrated with aq. 0.1% TFA/20%

$CH_3CN$, at 2.0 ml/min., $\lambda = 215nm$, full scale = 0.05. The principal peak eluted isocratically at 47.61 minutes.

The bulk of product was isolated using two 2.12 x 25 cm Vydac $C_{18}$ reverse phase columns in series, in aq. 0.1% TFA/21-26% $CH_3CN$ as a linear gradient over two hours at 10 ml/min. The main peak was collected, over several runs, and the combined analysis revealed $[M+H]^+ = 3116.8$, which corresponds nicely with the theoretical mass for $C_{135} H_{222} N_{44} O_{35} S_3 = 3115.61$, calculated on the basis of Carbon = 12.0000.

### B. Preparation of the Cyclic Disulfide:

(i) $K_3Fe(CN)_6$ Oxidation:

An aliquot of the linear peptide from A. above (6.9 mg 2.085 $_m$mole) was dissolved in degassed, distilled water (7 ml) at 23°C to give a clear, colorless solution of pH 3.4, and approximate concentration of 0.33 mmolar. The pH was adjusted to 8.5 by addition of 0.1N $NH_4OH$, and the solution was blanketed with $N_2$. An aliquot of this solution was analyzed on a 0.46 x 25 cm Vydac $C_{18}$ column in aq. 0.1% TFA/22% $CH_3CN$ $\lambda = 215nm$, A = 0.05, 2 ml/min. Peaks at 10.88 minutes and 7.16 minutes were noted. The 7.16 minute peak is the oxidized disulfide compound.

Freshly prepared aq. 0.01M $K_3Fe(CN)_6$ aqueous solution (0.417 ml, 4.17 $\mu$mole) was added, and, after 50 minutes of reaction time, an aliquot of the solution analyzed. All of the 10.88 minute material had disappeared and the 7.16 minute peak was greatly enhanced.

Aqueous 10% acetic acid was then added to, bring the pH to 4.1 which resolubilized a slight precipitate in the reaction. The sample was then concentrated to 1.0 ml. The cyclized product was isolated by chromatography on two 2.12 x 25.0 cm Vydac $C_{18}$ columns in series. The 1.0 ml sample was injected and the product eluted over 60 minutes by a gradient of aq. 0.1% TFA/21% $CH_3CN$-25% $CH_3CN$. A very sharp peak eluting at 47.75 minutes was collected, and evaporated, followed by lyophilization to give a colorless powder. FAB-MS. gave:

$[M+H]^+ = 3114.9$ and

$[M+Na]^+ = 3136.4$, which corresponds nicely with the calculated mass of 3113.6 for $C_{135} H_{220} N_{44} O_{35} S_3$.

(ii) Air Oxidation:

An aliquot of the linear 28mer (52 mg) from A. above was dissolved in 0.1% TFA/20% $CH_3CN$ (107 ml, 0.1 $\mu$moles/ml) at 23°C, and the solution allowed to stand at room temperature, exposed to the atmosphere.

Analytical chromatography of aliquots was conducted at 0 hours, 24 hours, 48 hours, 4 days, 7 days, 9 days, 12 days, 15 days. By 15 days, all of the linear material was converted to cyclic material. The clear, colorless solution was evaporated to about 2.4 ml, and the product isolated from two 2.12 x 25 cm Vydac $C_{18}$ reverse phase columns in series: 10 ml/min., $\lambda = 215nm$, A = 3, using a gradient of 0.1% TFA/20% $CH_3CN$ - 25% $CH_3CN$ over 60 minutes. A major peak of U.V. absorbing material eluting at 47 minutes was collected and evaporated, followed by lyophilization. The sample was redissolved in water (2.0 ml) and relyophilized to give a colorless powder (19.5 mg.).

An aliquot was analyzed on Vydac $C_{18}$ and co-injected with cyclic disulfide prepared as in B (i) above.

The sample was rechromatographed on two 2.12 x 25 cm reverse phase Vydac $C_{18}$ columns in series as before and the single peak eluting at 46.51 minutes was isolated, and the purity confirmed by analytical hplc. Amino acid analysis confirmed the correct amino acid composition for the hybrid peptide, cPND566:

| AMINO ACID | PREDICTED | FOUND |
|------------|-----------|-------|
| Thr | 3 | 2.79 |
| Ser | 1 | 0.94 |
| Pro | 3 | 3.14 |
| Gly | 3 | 3.00 |
| Ala | 1 | 1.02 |
| Val | 1 | 0.98 |
| Met | 1 | 0.41 |
| Ile | 2 | 1.96 |
| Leu | 1 | 1.05 |
| Tyr | 1 | 0.99 |
| Phe | 1 | 1.01 |
| His | 1 | 1.02 |
| Lys | 2 | 2.00 |
| Arg | 3 | 2.99 |
| Asn | 2 | 2.01 |
| I/2Cystine | 2 | 0 |

The low methionine level found is predicted and the absence of a Cys signal is also predicted, as these amino acids are known to degrade under the conditions of amino acid analysis employed.


EXAMPLE 2

Preparation of cPND244:

A. Preparation at the linear 21mer:

The 21mer was assembled on the MILLIGEN #9050 synthesizer, starting from Fmoc-L-Cys(Trt)-OPKA resin (MILLIGEN batch B090426, 0.081 meq./g, 2.47g = 0.200 meq), mixed with an equal volume of glass beads (SIGMA 150-212 $\mu$). Two 1 x 10 cm columns were charged with this resin and connected in series.

Reagents were Fmoc Pfp esters, except for Ser and Thr which were dihydrobenzotriazene (Dhbt) esters. The monomers were provided in four fold excess in NMP solvent. Side chain protection was as follows:

Thr, Ser    - Bu$^t$ = tert Butyl
Arg         - Mtr = 4- methoxy-2,3,5-trimethyl-phenylsulfonyl
Cys         - Trt = Trityl
His         - Boc = tert-butyloxycarbonyl

Double coupling was provided for Thr[3], Ser[6], Val[7], Pro[8], Asn[9], Pro[10], Ile[14], Gly[15], Pro[16], Phe[20]

The free amino derivatized resin, moist with $CH_2Cl_2$, was dried and suspended in 95% TFA/4% ethanadithiol/1% thioanisole (30 ml) and the peptide allowed to cleave from the resin for 4 hours at 25°C. The resin was filtered off and washed with 100% TFA (3 x 20 ml). The combined filtrate was evaporated and the TFA extracted by trituration of the viscous gum in ether (20 ml). The insoluble product was recovered by filtration, powdered by grinding in ether (3x4 ml), and dessicated to yield an almost colorless powder (443 mg).

About 50 $\mu$g of the powder was dissolved in 50 $\mu$l aq. 0.1% TFA/20% $CH_3CN$, and a 6 $\mu$l sample was analyzed on a Vydac $C_{18}$ reverse phase column (0.46 x 25.0 cm) equilibrated with aq. 0.1% TFA/25% $CH_3CN$, at 2.0 ml/min, $\lambda = 215$nm, full scale = 0.1. The principal peak eluted isocratically at 23.22 minutes. FAB-MS gave $[M+H]^+$ = 2185.5, and $[M+Na]^+$ = 2207.0, which corresponds nicely with the calculated mass of 2184.6 for $C_{94}H_{150}N_{28}O_{26}S_3$, calculated on the basis of Carbon = 12.0000..

The bulk of product was isolated using two 2.12 x 25 cm Vydac $C_{18}$ reverse phase columns in series, in aq. 0.1% TFA/26-31% $CH_3CN$ as a linear gradient over 60 minutes at 10 ml/min. Two main peaks were collected, one at 45.23 minutes, and one at 56.37 minutes. FAB-MS confirmed that the material eluting in each peak was related as a diastereomeric pair, due to partially racemized Fmoc-L-Cys(Trt)-OPKA starting resin, ie. $[M+H]^+$ = 2185.6, and $[M+Na]^+$ = 2206.6 for the first peak, and $[M+H]^+$ = 2185.6, and $[M+Na]^+$ = 2207.6 for the second.

B. Preparation of the Cyclic Disulfide:

The earlier eluting dithiol material (10 mg) was dissolved in degassed distilled water (15 mL) at 24°C, to form a clear colorless colution of pH 3.8, and approximate peptide concentration of 0.3 mmolar. Aq. 0.1N ammonium hydroxide was added to adjust the pH to 8.5, and the solution was quickly blanketed with N. Oxidation was followed by analytical BPLC using a 0.46 x 25.0 cm VYDAC $c_{18}$ reverse phase column. A $2\mu l$ sample was injected in aq. 0.1% TFA/26% acetonitrile, A = 0.05, monitored at 215 nm, 2.0 ml/min. At t = 0, a clean peak was observed at 16.16 minutes.

Freshly prepared $K_3Fe(CN)_6$, 0.01 M was added at a rate of about 0.2 $\mu$mole/min. By t = 28 min, about 560 $\mu$L had been added (about 60%) and the solution remained colorless. Analytical HPLC revealed the appearance of a new, predominant peak at 32.0 minutes with residual dithiol showing as a peak at 16.8 min. By t = 44 minutes, addtion of $K_3Fe(CN)_6$ was stopped, and analytical HPLC revealed only a clean peak at 33.0 minutes.

The reaction solution was acidified to pH 4.1 by addition of aq. 10% acetic acid, and the sample concentrated by evaporation to 2 mL. Using two 2.12 x 25 cm VYDAC $C_{18}$ columns in series, the solution was injected and eluted at 10 mL/min, by a linear gradient over 60 minutes from 28-32% acetonitrile in aq. 0.1% TFA. The major peak eluting at 45.81 minutes was collected and the eluate evaporated to about 6 mL, followed by lyophilization to give a colorless powder (8 mg). Analysis by Ellman assay was negative showing the complete conversion of the dithiol to the disulfide. FAB-MS gave: $[M+H]^+$ = 2185, $[M+Na]^+$ = 2207, which is in good agreement with the calculated mass for $C_{94}H_{150}N_{28}O_{26}S_3$ of 2184.6.

Amino acid analysis showed:

| Amino Acid | Calculated | Found |
|---|---|---|
| Thr | 1 | 1.06 |
| Ser | 1 | 1.01 |
| Pro | 3 | 3.06 |
| Gly | 3 | 2.96 |
| Ala | 1 | 1.000 |
| $1/2(Cys)_2$ | 2 | ----- |
| Val | 1 | 0.98 |
| Met | 1 | 0.6. |
| Ile | 1 | 0.93 |
| Leu | 1 | 1.00 |
| Phe | 1 | 0.99 |
| His | 1 | 0.99 |
| Arg | 1 | 0.93 |
| Asn | 2 | 1.98 |
| Nle | 1 | 0.95 |

EXAMPLE 3

Protocol for Inoculation of Animals with the Hybrid Peptide cPND566

The inoculum was formulated by emulsifying the hybrid peptide in Freund's adjuvant at a concentration of 300 $\mu$g/ml. Complete Freund's adjuvant was used for the first dose. Incomplete Freund's adjuvant was used for subsequent doses.

Rabbits were individually inoculated with three 300 $\mu$g doses of the formulated hybrid peptide. Each dose was injected intramuscularly. The doses were delivered four weeks apart (week 0, 4 and 8). The animals were bled at intervals of two weeks. Serum samples were prepared from each bleed to assay for the development of specific antibodies as described in the subsequent examples.

EXAMPLE 4

Analysis of Sera for Anti-cPND566 IgG Antibodies

Each serum sample was analysed by enzyme-linked immunoadsorbent assay (ELISA). Polystyrene microtiter plates were coated with 0.5 $\mu$g per well of the synthetic hybrid peptide in phosphate-buffered physiological saline (PBS) at 4°C. Each well was then washed with PBS containing 0.05% TWEEN-20 (PBS-T). Test serum, diluted serially in PBS-T, was added to the peptide-containing wells and allowed to react with the adsorbed peptide for one hour at 36°C. After washing with PBS-T, alkaline phosphatase-conjugated goat anti-human IgG was added to the test wells and was allowed to react for one hour at 36°C. The wells were then washed extensively in PBS-T. Each well received 0.1% p-nitrophenyl phosphate in 10% diethanolamine, pH 9.8, containing 0.5 mM $MgCl_2 \cdot 6H_2O$. The ensuing reaction was allowed to proceed at room temperature for 30 minutes, at which time it was terminated by the addiion of 3.0 N NaOH.

The greater the interaction of antibodies in the test serum with the peptide substrate, the greater is the amount of alkaline phosphatase bound onto the well. The phosphatase enzyme mediates the breakdown of p-nitrophenyl phosphate into a molecular substance which absorbs light at a wavelength of 405 nm. Hence, there exists a direct relationship between the absorbance at 405 nm of light at the end of the ELISA reaction and the amount of peptide-bound antibody.

All the rabbits inoculated with the hybrid peptide developed antibodies specifically capable of binding the peptide, as indicated by the anti-peptide titer of Table A, Example 5.

EXAMPLE 5

Analysis of Sera for Activity which Specifically Neutralizes HIV Infectivity

Virus-neutralizing activity was determined with an assay described by Robertson et al., J. Virol. Methods 20: 195-202 (1988). The assay measures specific HIV-neutralizing activity in test serum. The assay is based on the observation that MT-4 cells, a human T-lymphoid cell line, are readily susceptible to infection with HIV and, after a period of virus replication, are killed as a result of the infection.

The test serum was treated at 56°C for 60 minutes prior to the assay. This treatment is required to eliminate non-specific inhibitors of HIV replication. Heat-treated serum, serially diluted in RPMI-1640 cell culture medium, was mixed with a standard infection dose of HIV. The dose had been determined prior to the assay as containing the smallest quantity of virus required to kill all the MT-4 cells in the assay culture after a period of 7 days. The serum-virus mixture was allowed to interact for one hour at 37°C. It then was added to $1.0 \times 10^5$ MT-4 cells suspended in RPMI-1640 growth medium supplemented with 10% fetal bovine serum. The cultures were incubated at 37°C in a 5% $CO_2$ atmosphere for 7 days.

At the end of the incubation period, a metabolic dye, DTT, was added to each culture. This dye is yellow in color upon visual inspection. In the presence of live cells, the dye is metabolically processed to a molecular species which yields a blue visual color. Neutralized HIV cannot replicate in the target MT-4 cells and therefore does not kill the cells. Hence, positive neutralization is assessed by the development of blue color following addition of the metabolic dye.

All the rabbits inoculated with cPND566 hybrid peptide developed specific HIV infectivity-neutralizing activity, as indicated by the virus-neutralization titer of Table A.

## TABLE A

### Evaluation of cPND566 Hybrid Peptide Immunogenicity in Rabbits [a]

| Animal # | Week post-inoculation | Anti-hybrid peptide titer[b] | Virus-neutralization titer[c] |
|---|---|---|---|
| 1 | 0 | <20 | <10 |
|   | 2 | <20 | ND[d] |
|   | 4 | 100 | ND |
|   | 6 | 500 | 80 |
|   | 8 | 12,500 | ND |
|   | 10 | 12,500 | 640 |
|   | 12 | 12,500 | ND |
| 2 | 0 | <20 | <10 |
|   | 2 | <20 | ND |
|   | 4 | 100 | ND |
|   | 6 | 100 | 20 |
|   | 8 | 12,500 | ND |
|   | 10 | 12,500 | 80 |
|   | 12 | 12,500 | ND |

TABLE A cont'd

Evaluation of cPND566 Hybrid Peptide Immunogenicity in Rabbits[a]

| Animal # | Week post-inoculation | Anti-hybrid peptide titer[b] | Virus-neutralization titer[c] |
|---|---|---|---|
| 3 | 0 | <20 | <10 |
|   | 2 | <20 | ND |
|   | 4 | 100 | ND |
|   | 6 | 500 | 40 |
|   | 8 | 12,500 | ND |
|   | 10 | 12,500 | 40 |
|   | 12 | 12,500 | ND |
| 4 | 0 | <20 | <10 |
|   | 2 | <20 | ND |
|   | 4 | <20 | ND |
|   | 6 | 100 | 40 |
|   | 8 | 2,500 | ND |
|   | 10 | 2,500 | 320 |
|   | 12 | 12,500 | ND |

[a]Each animal was inoculated with 300 μg of the peptide per dose and described in Example 3. Doses were delivered intramuscularly at 0, 4 and 8 weeks.

[b]Reciprocal of end-point ELISA titer determined as described in Example 4.

[c]Reciprocal of end-point virus-neutralization titer determined as described in Example 5.

[d]ND: not done.

12

EXAMPLE 6

Preparation of cPND999:

The cyclic hybrid peptide:

cPND999:

H–Met Gly Thr Asn Leu Ser Val Pro Asn Pro Nle Cys Tyr Asn Lys Arg Lys Arg

[Seq. ID:14:]

```
                                                 Ile
                                 HO–Cys          His
                                 Gly             Ile
                                 Ile             Gly
                                 Ile             Pro
                                 Asn             Gly
                  Lys Thr Thr Tyr Phe Ala Arg
```

was prepared in an identical fashion to the preparation of cPND244. FAB.MS analysis of the purified peptide gave no meaningful data when the usual 3-nitrobenzyl alcohol matrix was employed. However, use of the reductive matrix, thioglycerol, gave a strog $[M+H]^+$ signal at 4035, which corresponds nicely to the average mass (4034) of the 36 mer linear dithiol. Since the isolated peptide was found to be completely devoid of sulfhydryl groups by Ellman assay [Ellman, G.L., Arch. Biochem. 82. 70 (1959)], the appearance of the dithiol (4034) after exposure to thiolglycerol indicates that the original product was the 36mer cyclic disulphide, whcih was confirmed by the correct amino acid analysis.

13

SEQUENCE LISTING

(2) INFORMATION FOR SEQ ID NO:1:

    (i) SEQUENCE CHARACTERISTICS:

        (A) LENGTH: 4 amino acids

        (B) TYPE: amino acid

        (D) TOPOLOGY: both

    (ii) MOLECULE TYPE: peptide

    (iii) HYPOTHETICAL: NO

    (v) FRAGMENT TYPE: internal

    (xi) SEQUENCE DESCRIPTION: SEQ ID NO:1:

Gly Pro Gly Arg
1

14

(2) INFORMATION FOR SEQ ID NO:2:

    (i) SEQUENCE CHARACTERISTICS:

        (A) LENGTH: 10 amino acids

        (B) TYPE: amino acid

        (D) TOPOLOGY: both

    (ii) MOLECULE TYPE: peptide .

    (xi) SEQUENCE DESCRIPTION: SEQ ID NO:2:

Met Gly Thr Asn Leu Ser Val Pro Asn Pro
1             5               10

(2) INFORMATION FOR SEQ ID NO:3:

    (i) SEQUENCE CHARACTERISTICS:

        (A) LENGTH: 21 amino acids

        (B) TYPE: amino acid

        (D) TOPOLOGY: both

    (ii) MOLECULE TYPE: peptide

    (xi) SEQUENCE DESCRIPTION: SEQ ID NO:3:

Met Gly Thr Asn Leu Ser Val Pro Asn Pro
1             5               10

Leu Gly Phe Phe Pro Asp His Gln Leu Asp Pro
             15             20

(2) INFORMATION FOR SEQ ID NO:4:

    (i) SEQUENCE CHARACTERISTICS:

        (A) LENGTH: 21 amino acids

        (B) TYPE: amino acid

        (D) TOPOLOGY: both

    (ii) MOLECULE TYPE: peptide

    (xi) SEQUENCE DESCRIPTION: SEQ ID NO:4:

Met Gly Gly Trp Ser Ser Lys Pro Arg Lys
1             5               10

Gly Met Gly Thr Asn Leu Ser Val Pro Asn Pro
                15           20

(2) INFORMATION FOR SEQ ID NO:5:

    (i) SEQUENCE CHARACTERISTICS:

        (A) LENGTH: 28 amino acids

        (B) TYPE: amino acid

        (D) TOPOLOGY: both

    (ii) MOLECULE TYPE: peptide

(ix) FEATURE:

    (A) NAME/KEY: Disulfide-bond

    (B) LOCATION: 11..28


(ix) FEATURE:

    (A) NAME/KEY: Modified-site

    (B) LOCATION: 28

    (D) OTHER INFORMATION: /label= -NH2


(xi) SEQUENCE DESCRIPTION: SEQ ID NO:5:


Met Gly Thr Asn Leu Ser Val Pro Asn Pro Cys Arg
1             5                10


Lys Arg Ile His Ile Gly Pro Gly Arg Ala
      15              20


Phe Tyr Thr Thr Lys Cys
        25


(2) INFORMATION FOR SEQ ID NO:6:


  (i) SEQUENCE CHARACTERISTICS:

    (A) LENGTH: 21 amino acids

    (B) TYPE: amino acid

    (D) TOPOLOGY: both


(ii) MOLECULE TYPE: peptide


(ix) FEATURE:

    (A) NAME/KEY: Disulfide-bond

    (B) LOCATION: 12..21

```
(ix) FEATURE:
     (A) NAME/KEY: Modified-site
     (B) LOCATION: 11
     (D) OTHER INFORMATION: /label= NORLEUCINE


(xi) SEQUENCE DESCRIPTION: SEQ ID NO:6:

Met Gly Thr Asn Leu Ser Val Pro Asn Pro Leu Cys His Ile Gly Pro
1               5                   10                  15

Gly Arg Ala Phe Cys
            20
```

(2) INFORMATION FOR SEQ ID NO:7:

```
     (i) SEQUENCE CHARACTERISTICS:
         (A) LENGTH: 13 amino acids
         (B) TYPE: amino acid
         (D) TOPOLOGY: both

     (ii) MOLECULE TYPE: peptide



     (xi) SEQUENCE DESCRIPTION: SEQ ID NO:7:

Thr Arg Pro Asn Asn Asn Thr Arg Lys Ser Ile His Ile
1               5                   10
```

(2) INFORMATION FOR SEQ ID NO:8:

```
     (i) SEQUENCE CHARACTERISTICS:
         (A) LENGTH: 13 amino acids
         (B) TYPE: amino acid
         (D) TOPOLOGY: both

     (ii) MOLECULE TYPE: peptide



     (xi) SEQUENCE DESCRIPTION: SEQ ID NO:8:

Tyr Thr Thr Gly Glu Ile Ile Gly Asp Ile Arg Gln Ala
1               5                   10
```

(2) INFORMATION FOR SEQ ID NO:9:

    (i) SEQUENCE CHARACTERISTICS:
        (A) LENGTH: 6 amino acids
        (B) TYPE: amino acid
        (D) TOPOLOGY: both

    (ii) MOLECULE TYPE: peptide

    (xi) SEQUENCE DESCRIPTION: SEQ ID NO:9:

    Arg Lys Arg Ile His Ile
    1          5

(2) INFORMATION FOR SEQ ID NO:10:

    (i) SEQUENCE CHARACTERISTICS:
        (A) LENGTH: 4 amino acids
        (B) TYPE: amino acid
        (D) TOPOLOGY: both

    (ii) MOLECULE TYPE: peptide

    (xi) SEQUENCE DESCRIPTION: SEQ ID NO:10:

    Tyr Thr Thr Lys
    1

(2) INFORMATION FOR SEQ ID NO:11:

    (i) SEQUENCE CHARACTERISTICS:
        (A) LENGTH: 11 amino acids
        (B) TYPE: amino acid
        (D) TOPOLOGY: both

    (ii) MOLECULE TYPE: peptide

    (xi) SEQUENCE DESCRIPTION: SEQ ID NO:11:

    Met Gly Gly Trp Ser Ser Lys Pro Arg Lys Gly
    1          5              10

(2) INFORMATION FOR SEQ ID NO:12:

    (i) SEQUENCE CHARACTERISTICS:
        (A) LENGTH: 10 amino acids
        (B) TYPE: amino acid
        (D) TOPOLOGY: both

    (ii) MOLECULE TYPE: peptide

    (xi) SEQUENCE DESCRIPTION: SEQ ID NO:12:

    Leu Gly Phe Phe Pro Asp His Gln Leu Asp
    1           5           10

(2) INFORMATION FOR SEQ ID NO:13:

    (i) SEQUENCE CHARACTERISTICS:
        (A) LENGTH: 13 amino acids
        (B) TYPE: amino acid
        (D) TOPOLOGY: both

    (ii) MOLECULE TYPE: peptide

    (ix) FEATURE:
        (A) NAME/KEY: Modified-site
        (B) LOCATION: 13
        (D) OTHER INFORMATION: /label= -NH2

    (xi) SEQUENCE DESCRIPTION: SEQ ID NO:13:

    Thr Lys Ile Ser Asp Phe Gly Ser Phe Ile Gly Phe Lys
    1           5           10

(2) INFORMATION FOR SEQ ID NO:14:

    (i) SEQUENCE CHARACTERISTICS:
        (A) LENGTH: 36 amino acids
        (B) TYPE: amino acid
        (D) TOPOLOGY: both

    (ii) MOLECULE TYPE: peptide

(ix) FEATURE:
    (A) NAME/KEY: Disulfide-bond
    (B) LOCATION: 12..36

(ix) FEATURE:
    (A) NAME/KEY: Modified-site
    (B) LOCATION: 11
    (D) OTHER INFORMATION: /label= NORLEUCINE


(xi) SEQUENCE DESCRIPTION: SEQ ID NO:14:

Met Gly Thr Asn Leu Ser Val Pro Asn Pro Leu Cys Tyr Asn Lys Arg
1                    5                  10               15

Lys Arg Ile His Ile Gly Pro Gly Arg Ala Phe Tyr Thr Thr Lys Asn Ile
        20                25              30

Ile Gly Cys
    35


(2) INFORMATION FOR SEQ ID NO:15:

    (i) SEQUENCE CHARACTERISTICS:
        (A) LENGTH: 21 amino acids
        (B) TYPE: amino acid
        (D) TOPOLOGY: circular

    (ii) MOLECULE TYPE: peptide


    (ix) FEATURE:
        (A) NAME/KEY: Peptide
        (B) LOCATION: 1
        (D) OTHER INFORMATION: /label= X1
            /note= "hydrogen,
            MetGlyGlyTrpSerSerLysProArgLysGly [Seq. Id:11:] or
            a portion thereof"

    (ix) FEATURE:
        (A) NAME/KEY: Peptide
        (B) LOCATION: 12
        (D) OTHER INFORMATION: /label= X2
            /note= "A BOND, OR LeuGlyPhePheProAspHisGlnLeuAsp
            [Seq.Id:12:] or a portion thereof, or 1-4 amino
            acids, optionally including a marker amino acid

# EP 0 547 689 A2

```
(ix) FEATURE:
      (A) NAME/KEY: Modified-site
      (B) LOCATION: 13
      (D) OTHER INFORMATION: /label= X3
            /note= "AN AMINO ACID BONDED TO X6, AND IS
            PREFERABLY CYSTEINE, IN WHICH CASE THE BOND IS A
            DISULFIDE BOND"

(ix) FEATURE:
      (A) NAME/KEY: Peptide
      (B) LOCATION: 14
      (D) OTHER INFORMATION: /label= X4
            /note= "AN AMINO ACID OR A PEPTIDE UP TO 25 AMINO
            ACIDS LONG"

(ix) FEATURE:
      (A) NAME/KEY: Peptide
      (B) LOCATION: 19
      (D) OTHER INFORMATION: /label= X5
            /note= "A BOND, AN AMINO ACID, OR A PEPTIDE UP TO
            25 AMINO ACIDS LONG"

(ix) FEATURE:
      (A) NAME/KEY: Modified-site
      (B) LOCATION: 20
      (D) OTHER INFORMATION: /label= X6
            /note= "AN AMINO ACID BONDED TO X3, AND IS
            PREFERABLY CYSTEINE, IN WHICH CASE THE BOND TO X3
            IS A DISULFIDE"

(ix) FEATURE:
      (A) NAME/KEY: Modified-site
      (B) LOCATION: 21
      (D) OTHER INFORMATION: /label= X7
            /note= "-OH, -NH2, AN AMINO ACID, OR A PEPTIDE UP
            TO 10 AMINO ACIDS LONG"


(xi) SEQUENCE DESCRIPTION: SEQ ID NO:15:


Xaa Met Gly Thr Asn Leu Ser Val Pro Asn Pro Xaa Xaa Xaa Gly Pro
1               5                   10                  15


Gly Arg Xaa Xaa Xaa
            20
```

## Claims

1. A hybrid immunogen comprising a T-cell epitope from HBSAg/p43 pre-S(1) and a cyclic HIV principal neutralizing determinant, cPND.

2. The hybrid immunogen of Claim 1 wherein the T-cell epitope is a peptide of between 10 and 20 amino acids in length, and the HIV PND is a cyclic peptide of between 5 and 40 amino acids in length, and the T-cell epitope and HIV PND are covalently linked through an optional intervening sequence of between 1 and 4 amino acids which may include a marker amino acid to form the hybrid immunogen.

22

3. The hybrid immunogen of Claim 2 wherein the T-cell epitope comprises the sequence: Met Gly Thr Asn Leu Ser Val Pro Asn Pro [Seq. ID.:2:].

4. The hybrid immunogen of Claim 3 wherein the cyclic HIV PND comprises the sequence: Gly Pro Gly Arg [Seq. ID:1:].

5. The hybrid immunogen of Claim 4 having the formula [Seq. Id:15:]:

   $X_1$ - Met Gly Thr Asn Leu Ser Val Pro Asn Pro-$X_2$ $X_3$ $X_4$ Gly Pro Gly Arg $X_5$ $X_5$ $X_7$

   wherein:

   $X_1$ is hydrogen
   Met Gly Gly Trp Ser Ser Lys Pro Arg Lys Gly [seq. Id.:11:], or a portion thereof;

   $X_2$ is a bond,
   Leu Gly Phe Phe Pro Asp His Gln Leu Asp [Seq. Id. 12:], or a portion thereof, or any sequence of 1 to 4 amino acids optionally including norleucine;

   $X_3$ is an amino acid which is bonded to $X_5$;

   $X_4$ is 1 to 25 amino acids long, selected from the sequence of amino acids found in any of the common isolates of HIV gp120, amino terminal and adjacent to the Gly Pro Gly Arg [Seq. Id.:1:] sequence;

   $X_5$ is a bond or 1 to 25 amino acids long, selected from the sequence of amino acids found in any of the common isolates at HIV gp120, carboxy-terminal and adjacent to the Gly Pro Gly Arg [Seq.Id.:1:] sequence;

   $X_5$ is an amino acid which is bonded to X3;

   $X_7$ is hydroxyl,
   -NH2, or
   between 1 and 10 amino acids

6. The hybrid immunogen of Claim 5 wherein $X_3$ and $X_5$ are cysteine, and they are bonded to each other though a disulfide bond;
   $X_1$ is hydrogen;
   $X_2$ is Nle;
   $X_4$ and $X_5$ are as defined in Claim 5; and
   $X_7$ is -OH or -NH$_2$.

7. The hybrid immunogen of Claim 6 having the formula:

```
H—Met Gly Thr Asn Leu Ser Val Pro Asn Pro

Cys Arg Lys Arg Ile His Ile Gly Pro Gly Arg Ala Phe Tyr Thr Thr Lys Cys—NH2,

[Seq. ID:5:]
```

```
H—Met Gly Thr Asn Leu Ser Val Pro Asn Pro Nle Cys His Ile Gly Pro Gly Arg Ala Phe Cys—OH, or
[Seq. ID.:6:]
```

```
H—Met Gly Thr Asn Leu Ser Val Pro Asn Pro Nle Cys Tyr Asn Lys Arg Lys Arg
                                                                        Ile
[Seq. ID:14:]                              HO—Cys              His
                                           Gly                 Ile
                                           Ile                 Gly
                                           Ile                 Pro
                                           Asn                 Gly
                                           Lys Thr Thr Tyr Phe Ala Arg
```

8. The use of a hybrid immunogen as claimed in Claim 1 for the manufacture of a medicament for inducing anti-HIV PND, anti-HIV, or HIV neutralizing immune response.

23

9.  A composition comprising the hybrid immunogen as claimed in Claim 1 and a pharmaceutically acceptable carrier.